# EUROPEAN PATENT SPECIFICATION

(11) **EP 1 411 986 B1**
(45) Date of publication and mention of the grant of the patent: **12.03.2008**
(21) Application number: 02762383.4
(22) Date of filing: 22.07.2002
(51) Int. Cl.: A61K 47/36, A61K 31/353

(54) **COMPOSITION COMPRISING EPIGALLOCATECHIN GALLATE**
ZUSAMMENSETZUNG ENTHALTEND EPIGALLOCATECHIN-GALLAT
COMPOSITION COMPRENANT DE L'EPIGALLOCATECHIN GALLATE

(30) Priority: 30.07.2001 EP 01118246
(43) Date of publication of application: 28.04.2004
(73) Proprietor: DSM IP Assets B.V., 6411 TE Heerlen (NL)
(72) Inventor: CHEN, Chyi-Cheng, CH-4102 Binningen (CH); LEUENBERGER, Bruno, CH-4123 Allschwil (CH)
(74) Representative: Teipel, Stephan
(86) International application number: PCT/EP2002/008127
(87) International publication number: WO 2003/011339

(56) References cited:
- EP-A- 0 573 682
- EP-A- 0 742 012
- WO-A-00/57875
- WO-A-01/49285
- WO-A-01/72318
- DE-A- 19 627 344
- DATABASE CA [Online] CHEMICAL ABSTRACTS SERVICE, COLUMBUS, OHIO, US; NAGATO, YUKIKO ET AL: "Immunostimulators in food and feed" retrieved from STN Database accession no. 129:342965 CA XP002221410 & JP 10 279486 A (TAIYO KAGAKU CO., LTD., JAPAN) 20 October 1998 (1998-10-20)
- DATABASE CA [Online] CHEMICAL ABSTRACTS SERVICE, COLUMBUS, OHIO, US; OOKUBO, TSUTOMU ET AL: "Bactericidal compositions for control bacteria in livestock" retrieved from STN Database accession no. 125:56925 CA XP002221411 & JP 08 099884 A (TAIYO KAGAKU KK, JAPAN) 16 April 1996 (1996-04-16)
- DATABASE WPI Section Ch, Week 199542 Derwent Publications Ltd., London, GB; Class B05, AN 1995-325471 XP002221412 & JP 07 223941 A (NIPPON HAM KK), 22 August 1995 (1995-08-22)
- DATABASE WPI Section Ch, Week 199423 Derwent Publications Ltd., London, GB; Class B02, AN 1994-186358 XP002221413 & JP 06 122626 A (TAIYO KAGAKU KK), 6 May 1994 (1994-05-06)
- PATENT ABSTRACTS OF JAPAN vol. 014, no. 086 (C-0690), 19 February 1990 (1990-02-19) & JP 01 299224 A (ITOUEN:KK), 4 December 1989 (1989-12-04)
- DATABASE WPI Section Ch, Week 199130 Derwent Publications Ltd., London, GB; Class B02, AN 1991-219271 XP002221414 & JP 03 141220 A (TSUMURA & CO), 17 June 1991 (1991-06-17)

## Description

The present invention relates to a composition in the form of a powder and/or granules, which contain as principal components (-)-epigallocatechin gallate (hereinafter: EGCG), together with a polysaccharide.

EGCG is the most interesting compound among group of polyphenols contained in the leaves of the green tea plant *Camellia sinensis* because it exhibits a strong antioxidant effect. Furthermore, it has been demonstrated that EGCG has an antimutagenic effect, an antibacterial effect and also a beneficial effect on cholesterol level in blood. EGCG may be obtained by procedures described in, e.g., European patent application no. 1077 211 A2. EGCG is normally produced in crystalline powder form that has a very poor flowability. The poor powder flow renders the crystalline powder difficult for use in tablet formulation and other applications that require the powder to be free flowing.

It has now been found that a composition containing EGCG together with polysaccharides may be obtained in the form of a powder or of granules with improved flowability.

Thus, in one aspect, the present invention relates to a composition in the form of a powder or granules comprising:
(a) (-)-epigallocatechin gallate, and
(b) a polysaccharide.

In a further aspect, the present invention relates to a method of producing the composition of the present invention.

In still another aspect, the invention is concerned with the use of polysaccharides, particularly pectin, for improving the flowability of EGCG powder.

Examples of polysaccharides for use in the present invention are pectin, alginates, starch, cellulose derivatives such as hydroxypropyl methyl cellulose and carboxymethyl cellulose, carrageenan, agar, gum arabic, guar gum, xanthan gum and mixtures thereof. The preferred polysaccharide is pectin or alginate, most preferred is pectin.

Pectin is a polysaccharide and described for example in the book entitled Industrial Gums, third edition, Academic Press, Inc., 1993, pages 257ff. Commercial pectins are generally produced from either citrus peel or apple pomace. Other possible sources are sugarbeet, sunflower and mango. Preferred pectins to be used within the scope of the present invention are citrus pectins, which generally have lighter color than apple pectins and, thus, do not impart significant color to the granule product.

The polysaccharide is preferably used in quantities within the range of about 0.1% to about 5 % by weight, based on the total weight of the composition. If pectin is used as the polysaccharide, it is preferably used in quantities of about 0.1% to about 1 % by weight, based on the total weight of the composition. If alginate is used as the polysaccharide, it is preferably used in quantities of about 0.1 % to about 1 % by weight, based on the total weight of the composition. If starch is used as the polysaccharide, it is preferably used in quantities of about 0.1 % to about 5 % by weight, based on the total weight of the composition.

The composition of this invention may be produced by any method known *per se* for the production of powders or granules. Preferred are fluidized-bed granulation, high-shear granulation, extrusion, spray-drying and wet granulation.

For obtaining the composition of the present invention by spray-drying it is convenient to prepare an aqueous slurry of all the components. The slurry has preferably a solid content of about 10 to 70% by weight, and preferably about 25 to 50% by weight. The slurry is then spray-dried in a manner known per se.

For obtaining the composition of the present invention by fluidized-bed granulation it is convenient to use a known fluidized-bed granulating apparatus which comprises a fluidized-bed drying device fitted with spray means. Preferably the EGCG forms the fluidized bed, the fluidized bed being fluidized by air or an inert gas, e.g. nitrogen. The polysaccharide or polysaccharides are dissolved in an appropriate amount of water and sprayed in the form of an atomized mist onto the fluidized particles in such a manner that the granulating and drying operations is accomplished in a single step. Alternatively, the polysaccharide or polysaccharides are mixed with EGCG and the fluidized bed being fluidized by air or an inert gas, e.g. nitrogen. An appropriate amount of water is sprayed in the form of an atomized mist onto the fluidized particles in such a manner that the granulating and drying operations is accomplished in a single step. The granulating process is continued until the desired-granule or powder is obtained. At the end of the granulation process, the granules may be sieved to fractionate the granules as to size. While the particle size is not narrowly critical to the invention it is, for practical purposes, preferably within 50 and 2000 micron, more preferably between 100 and 1000 microns.

The composition thus obtained may be further processed depending on the intended use of the EGCG or desired applications. For instance, the composition may be compressed into tablets with conventional tabletting methods and machinery. Optionally the powder or the granules may further be mixed with a lubricant or a mixture of lubricants and then compressed into tablets. If additional lubricant is used it is preferably selected from the group of stearic acid or the magnesium or calcium salt thereof, or glyceryl behenate 45 (Compritol 888 ATO), preferably in an amount of about 0.5 to 4% by weight, calculated to the total weight of the composition. Or the composition may be mixed with excipients. Examples for excipients are dextrinized sucrose (Di Pac sugar), microcrystalline cellulose or starch.

The invention is illustrated further by the following Examples.

### Example 1

EGCG powder as obtained by the procedures disclosed in EP 1 077 211 A2 may be used. A pectin solution is prepared by dissolving 27.3 g of pectin (Pectin USP, 8.4% moisture content, Danisco Ingredients, Denmark) in 1000 g of water. EGCG powder is placed in a Glatt Fluidized-Bed granulator (Model Uniglatt, Glatt GmbH, Germany) and sprayed with a fine mist of pectin solution. The granulation conditions are suitably as follows:
EGCG powder: 594 g
Pectin solution: 246.6 g
Pectin solution spraying rate: 6.7 g/minute
Inlet air temperature: 80 °C
Outlet air temperature: 40 °C

The granules leaving the apparatus will have a moisture content of about 0.2 % by weight, based on the granule weight.

### Example 2

A pectin solution was prepared by dissolving 5.82 g of pectin (Pectin USP/100, 8.96% moisture content, CP Kelco, Denmark) in 174.2 g of water. EGCG powder was placed in a Glatt Fluidized-Bed granulator (Model Uniglatt, Glatt GmbH, Germany) and sprayed with a fine mist of pectin solution. The granulation conditions were suitably as follows:
EGCG powder: 445.5 g
Pectin solution: 150 g
Pectin solution spraying rate: 6.7 g/minute
Inlet air temperature: 80 °C
Outlet air temperature: 40 °C

At the end of granulation, the granule was dried for about 5 minutes to a moisture content of about 0.2 % by weight, based on the granule weight. Sieve analysis gave the following particle size distribution.

| Particle Size Micron | > 850 | > 800 | > 600 | > 425 | > 250 | > 160 | > 125 | < 125 |
|---|---|---|---|---|---|---|---|---|
| % by weight | 1 | 0 | 6 | 15 | 26 | 28 | 9 | 13 |

Granulated EGCG was compared with EGCG powder (the starting material for granulation) for powder flowability using the Agway test. In that test, flowability is determined by placing 100 gram of the granules in a glass funnel with a 11-mm opening, which is sealed temporarily. The measurement is started by releasing the seal. Flowability is determined as the time for the entire powder to flow through the funnel in seconds per 100 g of granule.
The time for a 100-g sample of the product obtained flowing through an 11 mm opening Agway funnel was 9.2 seconds. EGCG powder did not flow whereas granulated EGCG showed an acceptable flowability for use in tabletting and improved handling properties in various food and beverage applications.

## Claims

1. A composition in the form of a powder or granules comprising:
a) (-)-epigallocatechin gallate, and
b) a polysaccharide.

2. A composition according to claim 1, wherein the polysaccharide is pectin or alginate.

3. A composition according to claim 1, wherein the polysaccharide is pectin.

4. A composition according to any one of the claims 1-3, wherein the polysaccharide is present in quantities within the range of about 0.1 % to about 5% by weight, based on the total weight of the composition.

5. A composition according to claim.3, wherein the pectin is present in quantities within about 0.1 % to about 1 % by weight, based on the total weight of the composition.

6. A process for preparing a composition according to any one of the claims 1-5, which comprises preparing an aqueous slurry of all the components, preferably having a solid content of about 10 to 70% by weight, and preferably about 25 to 50% by weight and spray-drying the slurry in a manner known per se.

7. A process for preparing a composition according to any one of the claims 1-5, which comprises forming a fluidized bed of (-)-epigallocatechin gallate, with or without polysaccharide, within a fluidized-bed drying device fitted with spray means, said fluidized bed being fluidized by air or an inert gas, and spraying an aqueous solution of a polysaccharide or only with water in the form of an atomized mist onto the fluidized particles until the desired granule or powder is obtained.

8. A process as in claim 7 wherein the polysaccharide is pectin or alginate.

9. A process as in claim 7 wherein the polysaccharide is pectin.

10. The use of a polysaccharide for improving the flowability of EGCG powder.

11. The use of pectin for improving the flowability of EGCG powder.

## Patentansprüche

1. Zusammensetzung in Form eines Pulvers oder von Körnchen, umfassend:
a) (-)-Epigallocatechingallat und
b) ein Polysaccharid.

2. Zusammensetzung nach Anspruch 1, wobei das Polysaccharid Pectin oder Alginat ist.

3. Zusammensetzung nach Anspruch 1, wobei das Polysaccharid Pectin ist.

4. Zusammensetzung nach einem der Ansprüche 1 bis 3, wobei das Polysaccharid in Mengen innerhalb des Bereiches von etwa 0,1 bis etwa 5 Gew.-% vorliegt, bezogen auf das Gesamtgewicht der Zusammensetzung.

5. Zusammensetzung nach Anspruch 3, wobei das Pectin in Mengen zwischen etwa 0,1 und etwa 1 Gew.-% vorliegt, bezogen auf das Gesamtgewicht der Zusammensetzung.

6. Verfahren zur Herstellung einer Zusammensetzung nach einem der Ansprüche 1 bis 5, umfassend das Herstellen einer wässerigen Aufschlämmung aller Komponenten, bevorzugt mit einem Feststoffgehalt von etwa 10 bis 70 Gew.-% und bevorzugt etwa 25 bis 50 Gew.-%, und das Sprühtrocknen der Aufschlämmung in einer an sich bekannten Weise.

7. Verfahren zur Herstellung einer Zusammensetzung nach einem der Ansprüche 1 bis 5, umfassend das Bilden eines Fließbettes aus (-)-Epigallocaiechingallat mit oder ohne Polysaccharid innerhalb einer Fließbetttrocknungsvorrichtung, ausgestattet mit Sprühmitteln, wobei das Fließbett durch Luft oder ein inertes Gas fluidisiert wird, und das Sprühen einer wässerigen Lösung eines Polysaccharids oder nur Wasser in Form eines Sprühnebels auf die fluidisierten Teilchen, bis das gewünschte Körnchen oder Pulver erhalten wird.

8. Verfahren nach Anspruch 7, wobei das Polysaccharid Pectin oder Alginat ist.

9. Verfahren nach Anspruch 7, wobei das Polysaccharid Pectin ist.

10. Verwendung eines Polysacchharids zum Verbessern der Fließfähigkeit von EGCG-Pulver.

11. Verwendung von Pectin zum Verbessern der Fließfähigkeit von EGCG-Pulver.

## Revendications

1. Composition sous la forme d'une poudre ou de granulés comprenant :
a) du gallate de (-)-épigallocatéchine, et
b) un polysaccharide.

2. Composition selon la revendication 1, dans laquelle le polysaccharide est de la pectine ou de l'alginate.

3. Composition selon la revendication 1, dans laquelle le polysaccharide est de la pectine.

4. Composition selon l'une quelconque des revendications 1 à 3, dans laquelle le polysaccharide est présent dans des quantités situées au sein de la plage d'environ 0,1 % à environ 5 % en poids, en se basant sur le poids total de la composition.

5. Composition selon la revendication 3, dans laquelle la pectine est présente dans des quantités d'environ 0,1 % à environ 1 % en poids, en se basant sur le poids total de la composition.

6. Procédé de préparation d'une composition selon l'une quelconque des revendications 1 à 5, qui comprend la préparation d'une suspension épaisse aqueuse de tous les composants, de préférence ayant une teneur en solides d'environ 10 à 70 % en poids, et de préférence environ 25 à 50 % en poids et le séchage par pulvérisation de la suspension épaisse d'une manière connue en soi.

7. Procédé de préparation d'une composition selon l'une quelconque des revendications 1 à 5, qui comprend la formation d'un lit fluidisé de gallate de (-)-épigallocatéchine, avec ou sans polysaccharide, au sein d'un dispositif de séchage de lit fluidisé équipé d'un moyen de pulvérisation, ledit lit fluidisé étant fluidisé par de l'air ou un gaz inerte, et la pulvérisation d'une solution aqueuse d'un polysaccharide ou seulement avec de l'eau sous la forme d'une brume atomisée sur les particules fluidisées jusqu'à l'obtention des granulés ou de la poudre souhaités.

8. Procédé selon la revendication 7, dans lequel le polysaccharide est de la pectine ou de l'alginate.

9. Procédé selon la revendication 7, dans lequel le polysaccharide est de la pectine.

10. Utilisation d'un polysaccharide pour améliorer la fluidité de la poudre d'EGCG.

11. Utilisation de pectine pour améliorer la fluidité de la poudre d'EGCG.
